(19) **Europäisches Patentamt**
**European Patent Office**
**Office européen des brevets**

(11) **EP 1 673 445 B1**

(12) **EUROPEAN PATENT SPECIFICATION**

(45) Date of publication and mention
of the grant of the patent:
**28.11.2007 Bulletin 2007/48**

(51) Int Cl.:
*C12N 5/06* (2006.01)       *A61K 35/18* (2006.01)
*A61K 35/28* (2006.01)

(21) Application number: **04790426.3**

(22) Date of filing: **14.10.2004**

(86) International application number:
**PCT/EP2004/011570**

(87) International publication number:
**WO 2005/040360 (06.05.2005 Gazette 2005/18)**

(54) **BLOOD PRODUCTS FROM MESENCHYMAL STEM CELLS**

BLUTPRODUKTE AUS MESENCHYMALEN STAMMZELLEN

PRODUITS SANGUINS ISSUS DE CELLULES SOUCHES MESENCHYMATEUSES

(84) Designated Contracting States:
**AT BE BG CH CY CZ DE DK EE ES FI FR GB GR HU IE IT LI LU MC NL PL PT RO SE SI SK TR**

(30) Priority: **14.10.2003 US 510980 P**

(43) Date of publication of application:
**28.06.2006 Bulletin 2006/26**

(73) Proprietor: **Universitätsklinikum Hamburg-Eppendorf**
**20246 Hamburg (DE)**

(72) Inventors:
  • **LANGE, Claudia**
    **22529 Hamburg (DE)**
  • **GEHLING, Ursula**
    **22529 Hamburg (DE)**
  • **ZANDER, Axel, Rolf**
    **22359 Hamburg (DE)**

(74) Representative: **UEXKÜLL & STOLBERG**
**Patentanwälte**
**Beselerstrasse 4**
**22607 Hamburg (DE)**

(56) References cited:
**WO-A-99/61588          WO-A-03/070922**

  • PEI XUETAO: "Stem cell engineering:The new generation of cellular therapeutics" INTERNATIONAL JOURNAL OF HEMATOLOGY SUPPL.I, vol. 76, August 2002 (2002-08), pages 155-156, XP009042425

  • OSAWA M ET AL: "Long-term lymphohematopoietic reconstitution by a single CD34 low/negative hematopoietic stem cell" SCIENCE, AMERICAN ASSOCIATION FOR THE ADVANCEMENT OF SCIENCE,, US, vol. 273, no. 5272, 12 July 1996 (1996-07-12), pages 242-245, XP002097289 ISSN: 0036-8075

  • HU YING ET AL: "Transplantation of mesenchymal stem cells followed by G-CSF injection can reconstitute hematopoiesis of lethally irradiated BALB/C mice" BLOOD, vol. 98, no. 11 Part 2, 16 November 2001 (2001-11-16), page 316b, XP009042610 & 43RD ANNUAL MEETING OF THE AMERICAN SOCIETY OF HEMATOLOGY, PART 2; ORLANDO, FLORIDA, USA; DECEMBER 07-11, 2001 ISSN: 0006-4971

  • JIANG YUEHUA ET AL: "Pluripotency of mesenchymal stem cells derived from adult marrow" NATURE (LONDON), vol. 418, no. 6893, 4 July 2002 (2002-07-04), pages 41-49, XP001204372 ISSN: 0028-0836

  • REYES MORAYMA ET AL: "Origin of endothelial progenitors in human post-natal bone marrow" BLOOD, vol. 98, no. 11 Part 1, 16 November 2001 (2001-11-16), page 821a, XP009042605 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971

  • PEI X: "Who is hematopoietic stem cell: CD34+ or CD34-?" INTERNATIONAL JOURNAL OF HEMATOLOGY. DEC 1999, vol. 70, no. 4, December 1999 (1999-12), pages 213-215, XP009042521 ISSN: 0925-5710

SEQUENCE LISTING

<110> Universitätsklinikum Hamburg-Eppendorf

<120> BLOOD PRODUCTS FROM MESENCHYMAL STEM CELLS

<130> P 66936

<150> US 60/510,980
<151> 2003-10-14

<160> 48

<170> PatentIn version 3.1

<210> 1
<211> 19
<212> DNA
<213> Artificial

<220>
<223> Primer

<400> 1
ctgctccttg cccagtctg         19

<210> 2
<211> 21
<212> DNA
<213> Artificial

<220>
<223> Primer

<400> 2
gaatagctct ggtggcttgc a         21

<210> 3
<211>. 20
<212> DNA
<213> Artificial

<220>
<223> Primer

<400> 3
ccacgactgt cgtagcaggt          20

<210> 4
<211> 20
<212> DNA
<213> Artificial

<220>
<223> Primer

<400> 4
gctgttctgc aggtgaagag          20

<210> 5
<211> 20
<212> DNA
<213> Artificial

<220>
<223> Primer

<400> 5 20
gctgttctgc aggtgaagag          20

<210> 6
<211> 19
<212> DNA
<213> Artificial

<220>
<223> Primer

<400> 6
cctctactgc agacacaca           19

<210> 7
<211> 18
<212> DNA
<213> Artificial

<220>
<223> Primer

<400> 7 18
ggcttgagcc actgcagc           18

<210> 8
<211> 19
<212> DNA

<213> Artificial

<220>
<223> Primer

<400> 8 19
cctcaatggt acagcgtgc          19

<210> 9
<211> 20
<212> DNA
<213> Artificial

<220>
<223> Primer

<400> 9
gggttgcaaa tccagagaaa          20

<210> 10
<211> 19
<212> DNA
<213> Artificial

<220>
<223> Primer

<400> 10
ggacaattcc acacagtgg          19

<210> 11
<211> 19
<212> DNA
<213> Artificial

<220>
<223> Primer

<400> 11
ggagtaccat caccaagca          19

<210> 12
<211> 19
<212> DNA
<213> Artificial

<220>
<223> Primer

<400> 12
gtcagctgga gcgacgtca          19

<210> 13
<211> 19
<212> DNA
<213> Artificial

<220>

<223> Primer

<400> 13
ggagtcaaag gagaggctc          19

<210> 14
<211> 20
<212> DNA
<213> Artificial

<220>
<223> Primer

<400> 14
ccttcattct gaacaggcaa          20

<210> 15
<211> 19
<212> DNA
<213> Artificial

<220>
<223> Primer

<400> 15
ccttggcatt tgggtcatt          19

<210> 16
<211> 19
<212> DNA
<213> Artificial

<220>
<223> Primer

<400> 16
gagtgagcct ctccgtgta          19

<210> 17
<211> 19
<212> DNA
<213> Artificial

<220>
<223> Primer

<400> 17
gctcttcaga agctggcac          19

<210> 18
<211> 20
<212> DNA
<213> Artificial

<220>
<223> Primer

<400> 18

ccagatttgc cactgtgatg          20

<210> 19
<211> 20
<212> DNA
<213> Artificial

<220>
<223> Primer

<400> 19
ccacttctgg gagatgcgca          20

<210> 20
<211> 20
<212> DNA
<213> Artificial

<220>
<223> Primer

<400> 20
ccacactgct cagcacgaag          20

<210> 21
<211> 18
<212> DNA
<213> Artificial

<220>
<223> Primer

<400> 21
gcactgcgag gtcaacac          18

<210> 22
<211> 18
<212> DNA
<213> Artificial

<220>
<223> Primer

<400> 22
aggcacttgg caccattc          18

<210> 23
<211> 18
<212> DNA
<213> Artificial

<220>
<223> Primer

<400> 23
cagaaacgcc gagggtga          18

<210> 24

<211> 25
<212> DNA
<213> Artificial

<220>
<223> Primer

<400> 24
ttagaagagg tggaagttgg agtca        25

<210> 25
<211> 20
<212> DNA
<213> Artificial

<220>
<223> Primer

<400> 25
acaaccacca ccttatggcg        20

<210> 26
<211> 20
<212> DNA
<213> Artificial

<220>
<223> Primer

<400> 26
gcatgcactt tgacagctcc        20

<210> 27
<211> 18
<212> DNA
<213> Artificial

<220>
<223> Primer

<400> 27
cctgcagtcc ctttcgac        18

<210> 28
<211> 18
<212> DNA
<213> Artificial

<220>
<223> Primer

<400> 28
gcaactggtg aacggtaa        18

<210> 29
<211> 19
<212> DNA
<213> Artificial

<220>
<223> Primer

<400> 29
cgacttctca gaaggcaga        19

<210> 30
<211> 19
<212> DNA
<213> Artificial

<220>
<223> Primer

<400> 30
gggagacgca tagccttgt        19

<210> 31
<211> 19
<212> DNA
<213> Artificial

<220>
<223> Primer

<400> 31
ggtcaactca gttccagag        19

<210> 32
<211> 18
<212> DNA
<213> Artificial

<220>
<223> Primer

<400> 32
ctggtgcaca ggtaaaag        18

<210> 33
<211> 19
<212> DNA
<213> Artificial

<220>
<223> Primer

<400> 33
ggagcaaaca cagttggat        19

<210> 34
<211> 19
<212> DNA
<213> Artificial

<220>
<223> Primer

<400> 34
ggcatcagag actgtgctt          19

<210> 35
<211> 21
<212> DNA
<213> Artificial

<220>
<223> Primer

<400> 35
ggagatctgt gagaataggc t          21

<210> 36
<211> 20
<212> DNA
<213> Artificial

<220>
<223> Primer

<400> 36
catacatttc agcaggtgcg          20

<210> 37
<211> 25
<212> DNA
<213> Artificial

<220>
<223> Primer

<400> 37
tacttgtcca tcgtcatgga tccag          25

<210> 38
<211> 25
<212> DNA
<213> Artificial

<220>
<223> Primer

<400> 38
ctgtaacaga tgagatgctc caagg          25

<210> 39
<211> 19
<212> DNA
<213> Artificial

<220>
<223> Primer

<400> 39
ccacagactc agagagaac          19

<210> 40
<211> 18
<212> DNA
<213> Artificial

<220>
<223> Primer

<400> 40
ccttaacctg ggcagagc          18

<210> 41
<211> 19
<212> DNA
<213> Artificial

<220>
<223> Primer

<400> 41
ctggagagga tgttcctgt          19

<210> 42
<211> 18
<212> DNA
<213> Artificial

<220>
<223> Primer

<400> 42
gcttgaagtt gaccgggt          18

<210> 43
<211> 21
<212> DNA
<213> Artificial

<220>
<223> Primer

<400> 43
cctgtatata atcctcacca a          21

<210> 44
<211> 18
<212> DNA
<213> Artificial

<220>
<223> Primer

<400> 44
gctcatctgc aggcactg          18

<210> 45
<211> 19
<212> DNA

<213> Artificial

<220>
<223> Primer

<400> 45
gctgtatcat ggacca cct          19

<210> 46
<211> 18
<212> DNA
<213> Artificial

<220>
<223> Primer

<400> 46
gcttccatgg ctcatcct          18

<210> 47
<211> 20
<212> DNA
<213> Artificial

<220>
<223> Primer

<400> 47
ggagtttcca gaaatcataa          20

<210> 48
<211> 20
<212> DNA
<213> Artificial

<220>
<223> Primer

<400> 48
ggaatggcaa ttatctgcaa          20

**Description**

BACKGROUND

1. Technical Field

The present invention relates to blood products and methods of producing blood products from mesenchymal stem cells.

2. Background Information

Hematopoietic stem cells (HSCs) and Mesenchymal stem cells (MesSCs) are readily distinguishable cell populations based on cell surface markers. HSCs and MesSCs have previously been thought to give rise to distinct populations of cells and tissues.

HSCs were known to form blood cells, such as erythrocytes, myelocytes, platelets, dendritic cells, and lymphocytes. HSCs are also known as Bone Marrow stem cells (BMSC) and Marrow stem cells (MSC). HSCs have been derived from bone marrow and are identified as being non-adherent, CD34-positive and CD38-negative cells. Additionally, HSCs have been identified as Lin-negative and HLA DR-positive cells.

MesSCs form non-hematopoietic tissues such as bone, cartilage, tendon, fat, muscle, and neural cells. MesSCs are oligopotent progenitor cells located in bone marrow, blood, and other tissues that can differentiate into a variety of non-hematopoietic tissues including bone, cartilage, tendon, fat, muscle, and early progenitors of neural cells. MesSCs differentiate into different cell types depending on the interplay of a variety of environmental influences on the cells, including growth factors, and physical environment. MesSCs differ from HSCs in that MesSCs are identified as CD34-negative cells. MesSCs may also be identified as negative for CD45 and positive for CD105, CD59, CD90, CD13, and MHC I after at least one passage in culture.

Examples of MesSCs differentiation into non-hematopoietic tissues include MesSCs differentiation into mesoderm (fat, cartilage, bone, tendon, cardiac muscle and skeletal muscle; Pittinger, MF et al., 1999, Science, 248: 385-389; Jaiswal, N et al., 1997, J. Cell Biochem., 64(2):295-312; Shakibaei, M et al., 1997, Cell Biol. Int., 21(2):115-25.; Fukuda, K, 2001, Artif. Organs. 25(3):187-93.) and into ectoderm (neuronal cells, Deng et al. 2001, Biochem. Biophys. Res. Commun., 282(1):148-52.; Woodbury et al., 2000, J. Neurosci. Res., 61(4):364-70.). After transplantation of MesSCs into infarcted myocardium of rats, transplanted cells differentiated into cardiac muscle and were detected weeks later in regenerating heart tissue, as detected by a marker gene (Toma, C et al., 2002, Circulation, 105(1):93-8.). In other animal models, MesSCs were shown to differentiate into brain and spinal cord in injured or knockout animals, leading to improvement or healing of injured neural tissues. (Hofstetter et al., 1999, Proc. Natl. Acad. Sci. U.S.A., 99(4):2199-204.).

WO 99/61588 discloses a method of producing megacaryocytes *in vitro* comprising co-culturing human mesenchymal stem cells with CD34+ cells to induce the CD34+ cells to differentiate into megacaryocytes.

Some studies have suggested that MesSCs may be beneficial to support hematopoietic cells, such as in bone marrow transplantation, immunoregulation, and graft facilitation. However, MesSCs have not been cultured *in vitro* to form blood cells.

Advantages for using MesSCs to form blood products include the ability to greatly expand the MesSCs in culture after isolation. Unlike HSCs that do not propagate well in culture, MesSCs may be greatly expanded in culture and still retain the ability to differentiate into a plurality of blood products. For example, a small amount of bone marrow aspirate may provide enough MesSCs to differentiate into large numbers of cells to form the blood products of the present invention.

Thus, methods for providing blood products from MesSCs are desirable.

BRIEF SUMMARY

Accordingly, one object of the invention is to provide a method of producing blood products *in vitro* and for differentiating non-SV-40 transformed mesenchymal cells *in vitro,* in which

a) non-SV-40 transformed mesenchymal stem cells are isolated, which are CD34-negative, and which are negative for CD45 and positive for CD105, CD59, CD90, CD13 and MHC I after at least one passage in culture; and

b) said isolated non-SV40 transformed mesenchymal stem cells are cultured with the following growth factors added individually or in combinations thereof:

stem cell factor (SCF), thrombopoietin (TPO), flt-3 ligand (FL), interleukins, including interleukin-3 (IL-3) and interleukin-6 (IL-6), granulocyte-colony-stimulating factor (G-CSF), granulocyte-macrophage-colony-stimulating factor (GM-CSF), erythropoietin (Epo), vascular endothelial growth factor (VEGF), basic fibroblast growth factor (bFGF), hepatocyte growth factor (HGF), insulin-like growth factor (IGF), epidermal growth factor (EGF), leukaemia inhibitory factor (LIF), and hydrocortisone (HC),

for a time sufficient to produce at least one type of blood products.

Another object of the invention is to use the blood products obtained by the method of the invention for preparing a pharmaceutical composition for treating a patient in need thereof, as well as a method for preparing a pharmaceutical composition comprising the method of the invention for providing said blood product.

BRIEF DESCRIPTION OF THE DRAWINGS

FIG. 1 is a diagram of mesenchymal stem cell differentiation to form various blood products of the present invention;
FIG. 2 is a graph showing growth and doubling characteristics of the isolated mesenchymal stem cells of the present invention;
FIG. 3 is a graph showing blood cell counts after transplantation in mice with the MesSCs of the present invention; and
FIG. 4 is diagram showing the subpopulation of blood cells in transplanted mice.

DETAILED DESCRIPTION OF THE DRAWINGS AND THE PRESENTLY PREFERRED EMBODIMENTS

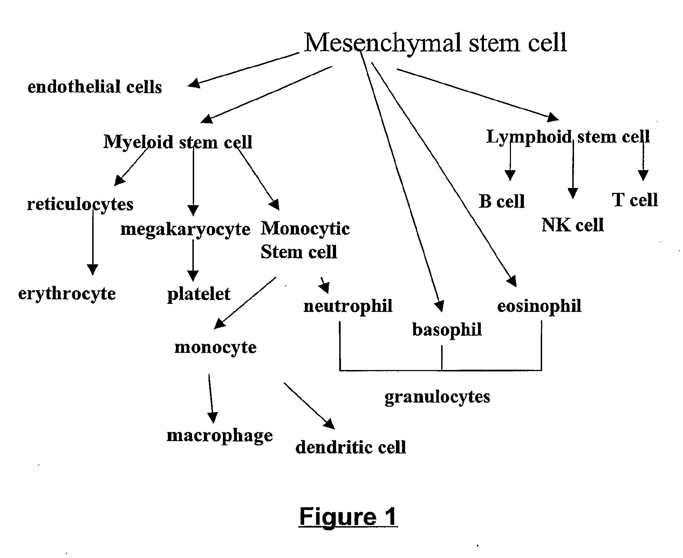

The present invention utilizes isolated, cultured mesenchymal stem cells (MesSCs) to produce blood products in the form of cells. Blood products produced with this method include those conventionally formed from hematopoietic stem cells. The terms "blood products" or "type of blood products" as used herein refers to all cells found in peripheral blood, including stem cells, immature, and mature cell populations. By way of example, the blood products include, but are not limited to, hematopoietic stem cells formed from cultured MesSCs, myeloid stem cells, endothelial cells, lymphoid stem cells, antigen presenting cells, erythroid cells, and megakaryocytes, and populations of cells derived therefrom. FIG. 1 diagrams the main developmental stages in blood cell formation. The process of blood cell formation is a continuum of proliferation and differentiation from the stem cells to the mature cells found in circulating blood. FIG. 1 illustrates exemplary blood products that may be formed from the cultured MesSCs. For example erythrocytes, macrophages and dendritic cells may all be derived from myeloid stem cells.

**Isolation of MesSCs**

MesSCs in accordance with the present invention can be identified by cell surface expression markers. Suitable MesSCs have a majority of the surface markers expressed as shown in Table I. In accordance with the present invention, non-SV40 transformed MesSCs are isolated, which are CD34-negative, and which are negative for CD45 and positive for CD105, CD59, CD90, CD13, and MHC I after at least one passage in culture.

**Table 1: Expression of surface markers on MesSCs**

| Marker | Expression |
|---|---|
| CD11a (LFA-1) | - |
| CD11b | - |
| CD13 | + |
| CD31 (PECAM) | - |
| CD34 | - |
| CD40 | - |
| CD40L | - |
| CD45 | - |
| CD49d (VLA-4) | + |
| CD58 (LFA-3) | + |

(continued)

| Marker | Expression |
|---|---|
| CD59 | ++ |
| CD80 (B7.1) | - |
| CD86 (B7.2) | - |
| CD90 (Thy 1) | + |
| CD105 (Endoglin) | + |
| CD117 (c-kit) | - |
| CD133 (AC133) | - |
| CXCR4 | - |
| CCR7 | - |
| CLA | - |
| ABCG2 | - |
| KDR (flk-1, VEGFR-2) | +/- (low) |
| SDF-1 | +/- intracellular |
| MHC I | + |
| MHC II | - |

In an embodiment of the present invention, described herein, the MesSCs used to produce blood products may be derived from bone marrow or blood, preferably bone marrow aspirates. However, the MesSCs may be derived from any source of cells known to one of skill in the art, including, but not limited to bone marrow, blood, dermis, and periostium. The term culturing as used herein includes growing MesSCs, expanding MesSCs, and differentiating MesSCs to form blood products. The term SV40 transformed as used herein refers to the transformation of cells *in vitro* into a cell line which is able to grow with unrestricted doublings, having an infinite life span using an SV-40 large T antigen to transform cells having a limited number of population doublings into a cell line, having infinite population doublings. Non-SV40 transformed cells have not been transformed with the SV-40 large T antigen. The term stem cell as used herein refers to a immature cell which is capable of giving rise to other cell types.

## Culturing, Expanding and Differentiating MesSCs

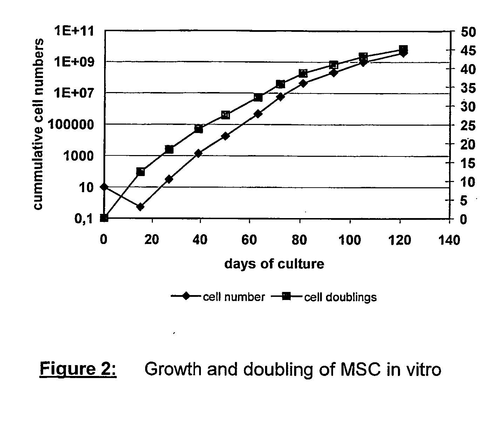

In a preferred embodiment of the present invention, the MesSCs are isolated from bone marrow aspirates using a Percoll® gradient, as described below. The growth characteristics of the isolated MesSCs are shown in FIG. 2.

After Percoll® gradient isolation, the MesSCs may be cultured and expanded *in vitro.* Following passage in culture, the MesSCs may be grown in the presence of differentiation media and growth factors to produce blood products, i.e. said isolated non-SV40 transformed MesSCs are cultured with the following growth factors added individually or in combinations thereof: stem cell factor (SCF), thrombopoietin (TPO), flt-3 ligand (FL), interleukins, including interieukin-3 (IL-3) and interleukin-6 (IL-6), granulocyte-colony stimulating factor (G-CSF), granulocyte-macrophage-colony stimulating factor (GM-CSF), erythropoietin (Epo), vascular-endothelial growth factor (VEGF), basic fibroblast growth factor (bFGF), hepatocyte growth factor (HGF), insulin-like growth factor (IGF), epidermal growth factor (EGF), leukaemia inhibitory factor (LIF), and hydrocortisone (HC). Alternatively, MesSCs may be grown in the presence of differentiation media and growth factors without passaging the MesSCs prior to differentiation. The blood cell products formed *in vitro* from the MesSCs will depend on the culture conditions selected for differentiation and are described in more detail below.

The cells isolated from the bone marrow may be used immediately, or alternatively, the cells may be stored for later expansion and/or differentiation. For example, freshly isolated bone marrow cells may be stored, preferably cryopreserved, as is routinely done in the field of bone marrow transplantation. The freshly isolated bone marrow comprises a mixture of cells, including MesSCs and HSC. The stored bone marrow cells may separated on a Percoll® gradient, isolated, cultured, and differentiated at a later time without substantial loss of proliferative or differentiation capacity. Isolated MesSCs may also be stored, preferably cryopreserved, after Percoll® centrifugation, and prior to *in vitro* culture. Cultured MesSCs may also be stored, preferably cryopreserved, in early passage after plating onto plastic in cell culture, such as P0 or P1 without substantial loss of proliferative or differentiation capacity. Potentially all blood products, at any step in the process from harvested bone marrow to differentiated blood products, may be stored for a period of time, prior to delivery to a patient. Storage of the blood products may be any type of storage known to one of skill in the art. Storage of blood products may be advantageous for many reasons, including, but not limited to, repeated administrations given to a patient, autologous donation for later use, characterization of cell populations and later use, and others.

In a preferred embodiment, the MesSCs are isolated from bone marrow aspirates. The bone marrow is obtained from autologous or allogeneic donors according to procedures known to one of skill in the art. Once the bone marrow is obtained from the donor, the MesSCs are isolated from the bone marrow aspirate using a Percoll® gradient.

In a preferred embodiment, a discontinuous gradient is prepared. Preparation of the gradient and separation of the MesSCs have been described in DE 103 36 152 (German Patent Application Serial No. 103 36 152.9, filed August 6, 2003) and in International Application No. PCT/EP2004/008865, filed August 6, 2004 (both applications being entitled "Purification procedure for mesenchymal stem cells").

In a preferred embodiment, Percoll® solution with the density 1.124 g/ml (Fa. Biochrom, Berlin, Germany) is used to prepare a discontinuous density gradient. Dilutions of the basic solution are prepared with PBS (Phosphate-buffered salt solution without $Ca^{++}$ and $Mg^{++}$, Gibco, Karlsruhe, Germany) according to the formula:

$$V[\%] = \frac{(D'-D\%) \times 10^2}{D''-D\%}$$

where:

D'    desired final density (g/ml)
D"    high basic density (g/ml)
D%    density of the iso-osmolale diluent (g/ml)
V%    volume-percent of the basic solution with high density.

Separation-solutions using this method can be prepared using a variety of densities, preferably with the following densities: 1.050, 1.063, 1.068, 1.077, 1.088 and 1.100 g/ml.

The bone marrow aspirates are diluted 1:1 with PBS and layered onto the Percoll® gradients and centrifuged for 20 min with 800 x g at room temperature (RT). The low-density cells are isolated from higher density red blood cells and mononuclear hematopoietic cells as describe below in Example 1. The low-density cells are selected for high proliferative potential, based on characterisation in further growth, phenotypic and differentiation assays. Alternatively, the MesSCs may be isolated by any method known to one of skill in the art.

The low-density cells comprising MesSCs are collected, washed twice in PBS, resuspended in growth medium comprising DMEM/low glucose (Gibco) supplemented with 10% preselected fetal calf serum (FCS) (BioWhittaker, Apen, Germany) and 1% penicillin/streptomycin (Gibco). The FCS was preselected by comparing growth characteristics for MesSCs grown in DMEM/LG + 10% FCS. The MesSCs were tested in passage P4 for the MesSCs' phenotype in FACS analysis and differentiation assays, including adipo-, osteo- and chrondrogeneic potential. The FCS was selected for promoting differentiation and for the greatest expansion of the test MesSCs. The isolated cells comprising MesSCs are counted in a Neubauer chamber. For initial seeding, $1 \times 10^7$ cells are seeded in tissue culture flasks with 25 $cm^2$ growth surface and grown in an incubator with 5% $CO_2$ and $\geq$ 97% humidity to confluency of about 90%. The isolated cells comprising MesSCs are preferably CD34-negative, plastic adherent, fibroblast-like cells. The isolated MesSCs grow through 4 to 10 passages, depending on the quality of the donor material, with an approximately constant doubling rate as shown in FIG. 2. Eventually, the proliferation potential decreases and the MesSCs cease growing. During the active proliferation phase, the isolated MesSCs display cell surface markers specific for MesSCs, including CD 105, CD 59, CD 90, CD 13 and MHC I. The MesSCs are negative for hematopoietic markers, including CD 34 and CD 45. For MesSCs differentiation into the plurality of blood products, various differentiation media and growth factors may be used. For example, a population of leukocytes expressing the pan leukocyte marker, CD 45, may be obtained within approximately two weeks of culture. The isolated MesSCs, which are CD34- and CD45-negative and at least CD90- and CD105-positive, may be grown in a differentiation media to produce a population of blood products comprising leukocytes, expressing CD45. The differentiation media for obtaining the CD45 positive cells includes IMDM as basal medium, supplemented with 10% FCS and 10% horse serum (HS), $1 \times 10^{-6}$ M HC mixed 1:1 with methylcellulose No. 4535 (CellSystems Biotech, containing the proliferation increasing cytokines SCF, IL-3, IL-6, G-CSF, GM-CSF. Epo is also added to the culture. Blood products comprising CD45 positive cells are obtained within 2 weeks of incubation. The medium for the cultured MesSCs is changed every second day.

To obtain blood products comprising myeloid stem cells, the MesSCs are first cultured in StemSpan (CellSystems Biotech) as serum free basal medium supplemented with SCF, TPO, GM-CSF and FL for two weeks to prestimulate the MesSCs. The medium for the cultured MesSCs is changed every second day. Thereafter, the MesSCs are harvested, resuspended in serum free methylcellulose No. 4436 (CellSystems Biotech, containing the proliferation increasing cytokines SCF, IL-3, IL-6, G-CSF, GM-CSF, and Epo) and incubated for an additional 2 weeks. The blood products formed may include monocytes/macrophages, indicated by CD 14 positive staining and granulocytes, including neutrophils, eosinophils and basophils, indicated by CD 16 positive staining. Additionally, BFU-E (burst forming unit-erythroid) structures may be formed from the myeloid culture conditions and identified by morphology and immunohistochemical staining with an antibody to glycophorine A.

For blood products comprising endothelial cells, the MesSCs were seeded in chamber-slides in StemSpan (StemCell Tech) as serum free basal medium supplemented with VEGF, bFGF and HGF and $10^{-6}$ M HC. The MesSCs were incubated for 3 weeks with medium changes every second day. Alternatively, MesSCs were prestimulated for two weeks in StemSpan supplemented with SCF, TPO, FL followed by differentiation for two weeks in StemSpan supplemented with VEGF, bFGF and IGF. The blood products comprising endothelial cells are identified by immunohistochemical staining for CD 31, CD 34, vWF (von Willebrand Factor), and KDR (also known as VEGFR2, Vascular Endothelial Growth Factor Receptor 2).

**Confirming Cell Types**

After differentiation, the blood products formed may be optionally cytochemically stained to confirm the cell type(s) present in the culture. Preferably, the blood product cell type will be confirmed using immunocytochemistry, more preferably, the cell type will be confirmed using cell surface marker expression of Cluster of Differentiation (CD) epitopes.

Within each lineage and between each lineage of blood product formed, antigens, such as CD antigens, are expressed differentially on the surface and in the cytoplasm of the cells in a given lineage. The expression of one or more antigens and/or the intensity of expression can be used to distinguish between maturational stages within a lineage and between lineages. The blood products formed from the differentiated cells may then be administered to a patient.

As described above, the blood products derived from MesSCs and the MesSCs are stained for cell surface marker expression to identify the blood products formed. In an embodiment of the present invention, the blood products are harvested, washed in PBS and centrifuged on slides for cytospin preparations. Alternatively, adherent blood products, such as endothelial cells, may be grown directly on chamber-slides for subsequent staining. The slides are air-dried, fixed for 10 minutes in ice-cold acetone. The acetone-fixed cells may be stored at -20°C until staining.

For staining, slides are warmed to RT and blocked with 5% normal goat serum in PBS for 10 min. After removal of the blocking solution, the cytospin preparations or chamber-slide preparations are incubated with titrated amounts of primary antibodies for 30 min at RT in a humidified chamber. The staining is followed by 2 washes in PBS for 10 min. each at RT and incubation with titrated amounts of fluorochrome-labeled secondary antibodies for 30 min. in a humidified chamber. Slides are washed twice with PBS and stained for 1 min. with DAPI to visualize the cell nucleus, followed by 2 washes with PBS for 10 min. A short wash of the slides with aqua dest. is followed by fixation for 5 min. in absolute ethanol and then air-drying. The slides are covered with fluoromount (Southern Biotech, Eching, Germany) and glass cover slipped and stored until analysis at 4°C. Alternative methods for staining are known to the skilled artisan or will become apparent from the instant description of the invention.

The analysis of the stained blood products is carried out using a high-power light and fluorescence microscope. As described above, the following staining, using the identified markers alone or in combinations thereof, has been observed for the identified blood products:

 a) myeloid stem cells positive for CD34, CD133, CD14, CD16, CD45;
 b) erythroid cells positive for Glycophorin A;
 c) megakaryocytic cells positive for CD41;
 d) endothelial cells positive for vWF, CD31, CD34, KDR;
 e) lymphoid cells positive for CD3 and

  i) T cells positive for CD4, CD8;
  ii) NK cells positive for CD56;
  iii) NK-T cells positive for CD3, NK1.1;
  iv) B cells positive for CD10; CD19; CD20;

 f) dendritic cells positive for CD80, CD86, MHC I, MHC II, CD1a, CD40.

**Delivery of blood products**

Delivery of the differentiated mesenchymal stem cells may be by injection, infusion or instillation of the blood products into the patient. The blood products may be injected, infused, or instilled directly into a desired target organ or alternatively the blood products may be injected intravenously, intra-arterially, or intraperitoneally. Any delivery method for cells, commonly known in the art, may be used for delivery of the blood products formed from MesSCs.

The blood products formed from the MesSCs cultured according to the method of the invention may be used to treat patients in need of a blood product. In certain embodiments, a therapeutically effective dose of blood products is delivered to the patient. An effective dose for treatment will be determined by the body weight of the patient receiving treatment. A therapeutic dose may be one or more administrations of the therapy. For example, where recovery of the blood cell numbers has been reached within about 2 weeks, one infusion of MesSCs may be sufficient for treatment. However, numerous applications of MesSCs blood products are not detrimental to the patients and may provide advantages over a single administration. An exemplary dose of a blood product may be about $1\text{-}50 \times 10^6$ per kilogram body weight.

The blood products used for treatment may be derived from autologous donor MesSCs or allogeneic donor MesSCs. Use of autologous MesSCs eliminates concerns regarding immune reactions provoked by the allogeneic cells. Additionally, repetitive administrations of autologous MesSCs are possible. Allogeneic MesSCs will be provided to the patient using matching criteria for organ transplantation commonly known to one of skill in the art. Allogeneic MesSCs derived from a compatible donor may also be advantageous for producing blood products for administration to a patient. Allogeneic blood products may be administered, for example, when the bone marrow in a patient who is in need of blood products may be a poor source of adequate numbers of usable stem cells because the patient may have received bone marrow toxic drugs or radiation or may have leukemia or bone marrow metastasis, when a patient may refuse or may not be able to consent to the harvesting of his/her own bone marrow cells, and when the bone marrow-derived stem cells from a compatible living-related or unrelated donor may be of superior quality and quantity compared to the recipient's own stem cells.

Examples of patients that may receive autologous blood products include cancer/leukemia patients in need of a bone marrow transplant and no compatible donor is available, cancer/leukemia patients undergoing chemotherapy, and patients developing thrombocytopenia after bone marrow transplant or chemotherapy. Additionally, patients who, after bone marrow transplantation or chemotherapy, have low erythrocyte or granulocyte counts may receive blood products derived from autologous MesSCs. Autologous blood product treatment may be advantageous for patients with allergic and autoimmune reactions.

Examples of patients that may receive allogeneic blood products include the same patients exampled for autologous blood product recipients listed above. Allogeneic cell preparations of MesSCs may be advantageous in that populations of specific cell types may be generated, screened, and stored. Thus providing a pool of specialized blood products including platelets, erythrocytes, leukocytes or specialized cells thereof, lymphocytes, and dendritic cells that are screened in advance of administration to the patient and certified to be, for example, disease free.

A further object of the invention is the use of at least one type of blood products obtained by the method of the invention for culturing isolated non-SV40 transformed mesenchymal stem cells *in vitro* with growth factors for a time sufficient to produce at least one type of blood products for preparing a pharmaceutical composition for treating of patients suffering from leukemia, thrombocytopenia, leukopenia, granulocytopenia, lymphocytopenia (such as HIV patients), aplastic anemia, and/or autoimmune disease with or without bone marrow involvement, patients after chemotherapy (including high-dose chemotherapy), total body irradiation or irradiation of single parts of the body (including irradiation of bones and organs) as well as patients with vascular, ischemic (including cardiac ischemia), and/or malignant disease. In particular, a further object of the invention is the use of at least one type of blood products obtainable by culturing isolated non-SV40 transformed mesenchymal stem cells *in vitro* with growth factors for a time sufficient to produce at least one type blood products for preparing a pharmaceutical composition for treating of patients suffering from anemia due to acute leukemia, due to chronic leukemia, due to osteomyelofibrosis, due to aplastic anemia, due to thalassaemia, due to sickle cell disease, due to loss of blood, e.g. after an accident, due to chemotherapy, due to medical drugs other than chemotherapy, due to radiation, and/or due to abuse of toxic compounds, and for treating of patients suffering from leukopenia due to acute leukemia, due to chronic leukemia, due to osteomyelofibrosis, due to aplastic anemia, due to thalassaemia, due to sickle cell disease, due to loss of blood, e.g. after an accident, due to chemotherapy, due to medical drugs other than chemotherapy, due to radiation, and/or due to abuse of toxic compounds, and for treating of patients suffering from thrombocytopenia due to acute leukemia, due to chronic leukemia, due to osteomyelofibrosis, due to aplastic anemia, due to thalassaemia, due to sickle cell disease, due to loss of blood, e.g. after an accident, due to chemotherapy, due to medical drugs other than chemotherapy, due to radiation, and/or due to abuse of toxic compounds, and for treating of patients suffering from vascular diseases, such as autoimmune vasculitis, arterial occlusive disorders, venous occlusive disease, and/or artherosclerosis, and for treating of patients suffering from ischemic diseases, such as coronary heart disease, stroke, acute renal failure, and/or claudicatio intermittens.

A further object of the invention is the use of at least one type of blood products obtained by the method of the invention for culturing isolated non-SV40 transformed mesenchymal stem cells *in vitro* with growth factors for a time sufficient to produce at least one type of blood products for preparing a pharmaceutical composition for diagnosis of cancers and metastasis, wherein said at least one type of blood products is labelled with radioactive compounds. When diagnosing cancers and metastasis, the type of blood products (or cells, respectively) are infused and detected in the patients body by means and methods well known to the skilled artisan.

Preferably, in connection with the above-mentioned use, said at least-one blood product comprises cells selected from the group consisting of myeloid stem cells, endothelial cells, lymphoid stem cells, dendritic cells, erythroid cells, and megakaryocytes.

The invention is further described and exemplified by the following examples.

**Examples:**

**Example 1 Isolation of MesSCs**

5 ml of each density 1.050, 1.063, 1.068, 1.077, 1.088 and 1.100 g/ml. of Percoll® (described above) were carefully layered onto each other into a 50 ml-tube. 10 ml of bone marrow were diluted with 10 ml PBS and carefully layered onto the density gradient. In parallel, as controls a) 1 ml bone marrow diluted with 1 ml PBS was layered onto 3 ml Ficoll with a density of 1.077 g/ml in a 15 ml-tube (designated as stem cells SC; conventional method to separate mononuclear cells as well as MesSCs) and b) each separate 1 ml bone marrow diluted with 1 ml PBS was layered onto 3 ml Percoll with a density of 1.068 g/ml (designated as LD = low density) or 1.077 g/ml (designated as MNC = mononuclear cells) in a separate 15 ml-tube.

All tubes were centrifuged for 20 min at room temperature with 800g without break. The plasma mixed with PBS was removed from the tubes and each fraction was collected into a separate tube. After washing the cells twice with PBS for 10 min with 400g, the erythrocytes contained in fractions F4-F6 (F1 = lowest density 1.050 g/ml, F6 = highest density 1,100 g/ml) were lysed with hemolysis buffer, the cells washed again in PBS, resuspended in cultivation-medium DMEM/LG (Dulbeccos's Modified Eagle Medium/low glucose, Gibco) +1% penicillin/streptomycin + 10% preselected fetal calf serum and counted with trypan-blue in a Neubauer chamber. For a growth area of 25 cm$^2$ 1 x 10$^7$ cells were seeded in 5 ml medium. After 3 days the nonadherent cells were washed off with PBS. Cells were fed every 3 days with fresh medium and incubated until reaching confluence of about 80-90% assessed by phase contrast microscopy. At this time point the culture was designated as P0.

For demonstrating the CFU-F (colony forming unit-fibroblast; as proof for proliferating activity of separate fractions of MesSCs) 10$^6$ cells of each fraction as well as LD and MNC control cells were seeded in a well of 6-well plates in 3 ml medium. The cells were incubated in an incubator with 37°C and 5% CO$_2$. After 3 days the nonadherent cells were washed off with PBS. Cells were fed every 3 days with fresh medium. The CFU-F were washed with PBS after an incubation period of 14 days and stained with 1% crystal violet.

For passaging the cells, the medium was removed, the plates washed once with PBS, incubated for 5 min with 0.25% trypsin-EDTA and the cells resuspended in medium and counted in a Neubauer chamber. 500 cells/cm$^2$ were seeded into a new tissue culture flask and designated as P1.

**Example 2 Isolation and Differentiation of MesSCs**

Aspirated bone marrow was diluted 1:1 with PBS (Gibco, Karlsruhe, Germany), layered onto ercoll®-solution (Biochrom, Berlin, Germany) with density 1.068 g/ml and centrifuged for 20 min with 800g at RT. Low-density cells were collected, washed twice in PBS, resuspended in growth medium DMEM/low glucose (Gibco) supplemented with 10% preselected FCS (BioWhittaker, Apen, Germany) and 1% penicillin/streptomycin (Gibco) and counted in a Neubauer chamber. For initial seeding, 1 x 10$^7$ cells were put in tissue culture flasks with 25 cm$^2$ growth surface and grown in an incubator with 5% CO$_2$ and ≥ 97% humidity to confluency of ca. 90%. Passaging was carried out by removing the culture medium, washing the growth surface with PBS and incubation with trypsin/EDTA (Gibco) for 5 min. The cells were resuspended in growth medium, counted and 500 cells/cm$^2$ seeded into new culture flasks.

For hematopoietic differentiation *in vitro* cultured MesSCs from the 2$^{nd}$ - 4$^{th}$ passage were used.

For differentiation into leukocytes, 1x10$^5$ cells were seeded on an area of 0.8 cm$^2$ in chamber slides and fed 3 times a week with differentiation medium. This medium contains a basic medium IMDM (Gibco) plus 10 % FCS plus 10 % HS (horse serum, both CellSystems Biotech, St. Katharinen, Germany) and 10$^{-6}$ M hydrocortisone (Sigma, Deisenhofen, Germany) and is mixed 1 : 1 with methylcellulose No. 4535 (CellSystems Biotech, containing the proliferation increasing cytokines SCF, IL-3, IL-6, G-CSF, GM-CSF) and Epo.

Formation of round cells was noted as early as day 5 in several of these cultures, at day 10 colonies of about 100 cells are produced. Colonies were picked and cytospun, the chamber slides were washed once with PBS, all slides air-dried, fixed for 10 min in ice-cold acetone and stored at - 20°C until staining. Alternatively, cells cultured in chamber slides may be typsinized for FACS analysis. The trypsinized cells were incubated with an anti-CD34 antibody or the respective isotype-matched immunoglobulin and analyzed on a Becton Dickinson FACScan.

For staining, slides were adapted to room temperature (RT) for some minutes and blocked with 5% normal goat serum in PBS for 10 min. After removal of the blocking solution the cytospins were incubated with titrated amounts of primary antibodies for 30 min at RT in a humidified chamber. For detection of myeloid, megakaryocytic and erythroid cells antibodies directed against CD133 (Miltenyi Biotec, Bergisch Gladbach, Germany), CD34, CD14, CD16, CD45, CD41 and Glycophorin A (all antibodies: Dako) were included. For each antibody the appropriate isotype control was carried out identically to the staining for antibodies.

The staining was followed by 2 washes in PBS for 10 min each at RT and incubation with titrated amounts of the fluorochrome-labeled secondary antibody Alexa 594 (Molecular Probes, Göttingen, Germany) for 30 min in a humidified chamber. Slides were washed 2x with PBS and stained for 1 min with DAPI (Sigma) to visualize the nucleus, followed by 2 washes with PBS for 10 min. A short wash with aqua dest. was followed by fixation for 5 min in absolute ethanol and air-drying. The slides were covered with fluoromount and glass cover slips and stored until analysis at 4°C.

The analysis was carried out at a high power light and fluorescence microscope. Using this approach, CD45-, CD133- and CD34-positive cells were detected in the clusters described above.

Example 3 Differentiation of MesSCs into Myeloid and Erythroid Cells

To obtain differentiation of MesSCs into myeloid and erythroid cells, $1 \times 10^5$ cells from the 2nd - 4th passage were seeded on a culture area of 2 cm$^2$ and pre-stimulated for 2 weeks with serum free differentiation medium consisting of the basal medium StemSpan (CellSystems) supplemented with the cytokines SCF (100 ng/ml), TPO (10 ng/ml), GM-CSF (10 ng/ml) and FL (50 ng/ml)(all from Tebu, Offenbach, Germany). Cells were fed 3 times a week.

Day 14 cells are taken out by heavy pipetting or trypsinization and cultivated in serum-free methylcellulose (CellSystems No. 4435, containing the proliferation increasing cytokines SCF, IL-3, IL-6, G-CSF, GM-CSF and erythropoietin). Following 14 days of cultivation single colonies and differentiated cells were taken out and examined after cytospin by morphology and immunochemistry with antibodies against the surface antigens of myeloid and erythroid characteristics as described in example 2.

Using this approach, CD34, CD133, CD14, CD16, CD45, CD41 and Glycophorin A positive cells were detected.

**Example 4 Differentiation of MesSCs into Endothelial Cells**

To obtain differentiation of MesSCs into endothelial cells, $1 \times 10^5$ cells from the 2nd - 4th passage were seeded on a culture area of 0.8 cm$^2$ and stimulated for 3 weeks with serum free differentiation medium including the basal medium StemSpan (CellSystems) supplemented with the cytokines SCF (100 ng/ml), VEGF (50 ng/ml), bFGF (10 ng/ml), HGF (50 ng/ml) (all from Tebu) and $10^{-6}$ M HC. Alternatively, MesSCs were prestimulated for two weeks in serum free medium supplemented with SCF (100 ng/ml), TPO (10 ng/ml) and FL (50 ng/ml), followed by differentiation in medium plus VEGF (50 ng/ml), bFGF (10 ng/ml), and IGF (10 ng/ml) for two weeks. Cells were fed 3 times a week. After finishing the differentiation slides were air-dried, fixed for 10 min in ice-cold acetone and stored at -20°C until staining.

Cells were stained according to the procedure described above with antibodies directed against vWF, CD31, CD34 and KDR. Positive cell staining was detected for CD34 and vWF or CD31 and KDR.

**Example 5 Transplantation of MesSCs**

MesSCs were transplanted into stem cell ablated mice to demonstrate the pluripotent potential of the MesSCs of the present invention.

MesSCs for transplantation were generated from adult male C57Bl/6J mice of the Ly 5.2 phenotype. Cells from femurs and tibiae were seeded in DMEM/Ham's F12 medium (generally regarded as medium insufficient supporting growth of hematopoietic stem cells) supplemented with 20% FCS, 1% penicillin/streptomycin and 1% glutamine. The FCS was selected for rapid growth of plastic adherent growing cells and for lack of support for growth of hematopoietic cells. Non-adherent cells were removed after 3 days and cultures fed twice a week until confluence. During early passages, up to P5, cells were seeded with $1 \times 10^5$ cells/ cm$^2$. Later passages were seeded with 1000 cells/ cm$^2$. Cells were passaged in culture up to 9 times over a period of about 6 months. Cells at P8 were determined to be negative for CD45 and CD34, positive for Sca-1, CD90 and CD59 and low positive for CD117 (c-kit).

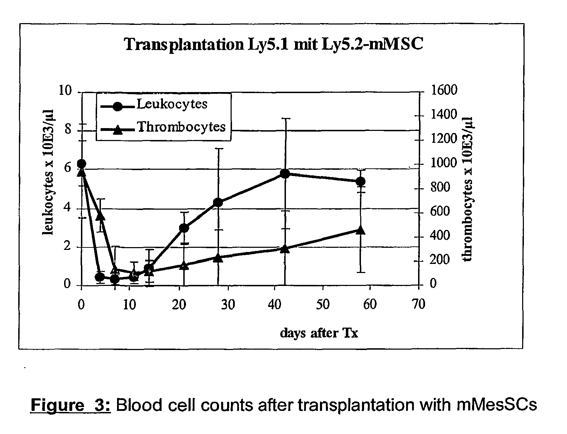

For MesSC transplantation, recipient C57Bl/6J mice of the Ly 5.1 phenotype were lethally irradiated with a 9.5 Gy dose of gamma irradiation and transplanted with $1 \times 10^6$ MesSCs cultured as described above. Blood cell counts and interim analyses of reconstitution with donor cells were performed from peripheral blood taken retroorbitally. The blood cell counts are shown in FIG. 3 and were performed using a Coulter® blood cell counter. Analysis of reconstitution was performed using cells labelled with fluorochrome-conjugated antibodies against a donor-antigen CD45.2 (Ly 5.2) and analyzed on a FACScan from Becton Dickinson to distinguish donor and recipient cells. The results of the reconstitution analysis are listed below in Table 2 where the percent reflects the percent of total cells that were of donor origin.

**Table 2: Recipient Reconstitution**

| Weeks | % Donor origin (+/- S.D.) |
|-------|---------------------------|
| 8 | $9.3 \pm 5.3$ % (0.1-19.3 %) |

(continued)

| Weeks | % Donor origin (+/- S.D.) |
|---|---|
| 15 | 6.9 ± 2.6 % (3.4-13.7 %) |
| 24 | 15.6 ± 6 % (10-24.5 %) |

Results show that mice can be reconstituted with MesSCs of the present invention leading to normal white blood cell counts after about 6 weeks and out of the danger zone for the acute risk of infection after about 3 weeks.

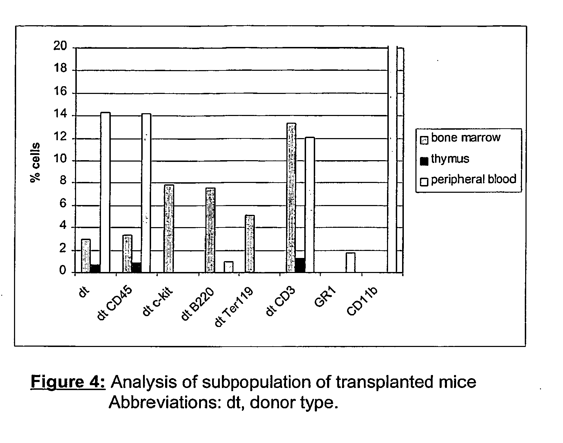

Analysis of cell composition of the individual tissues from MesSC-transplanted mice is shown for bone marrow, thymus and peripheral blood in Tables 3, 4 and 5, respectively. In general, the transplanted mice showed a low donor chimerism in bone marrow and thymus and up to 35% in peripheral blood (FIG. 4). Population analysis reveals that the donor cells mainly reconstituted the myeloid compartment and in part the lymphoid cells.

### Table 3: Bone Marrow Analysis

| | % donor type (dt) | % dt+ CD45+ | % dt+ c-kit+ | % dt+ B220+ | % dt+ Ter119+ | % dt+ CD3+ |
|---|---|---|---|---|---|---|
| Mouse 2 | 2.3 | Nd | 6 | 7.8 | 4.6 | 17.3 |
| Mouse 3 | 2.7 | 2.4 | 1.8 | 8.1 | 5.5 | 13.5 |
| Mouse 5 | 4.5 | 5 | 11.2 | 17.1 | 7.6 | 20.6 |
| Mouse 6 | 2.3 | 2.2 | 5.8 | 3.5 | 5.4 | 6.7 |
| Mouse 7 | 2.8 | 3.1 | 7.2 | 3.4 | 4.8 | 12.3 |
| Mouse 8 | 4 | 3.5 | 4.9 | 3 | 7.6 | 14.2 |
| Mouse 9 | 3.5 | 4 | 7.7 | 6.1 | 5.3 | 10.3 |
| Mouse 10 | 3.1 | 3.6 | 6.8 | 4.7 | 5.3 | 14.9 |
| Mouse 11 | 2 | 2 | 5 | 2.9 | 5 | 11.9 |
| Mouse 12 | 3.6 | 6.2 | 28.2 | 27.5 | 4.4 | 23.2 |
| Mouse 14 | 2.2 | 2.2 | 5 | 4.3 | 2.9 | 8.1 |
| Mouse 18 | 2.9 | 2.7 | 4.8 | 2.5 | 2.2 | 6.6 |
| Note: CD3-counts based on single cells in the gate | | | | | | |

### Table 4: Thymus Analysis

| | % donor type (dt) | % dt+ CD45+ | % dt+ CD3+ |
|---|---|---|---|
| Mouse 2 | 0.8 | 1.1 | 1.1 |
| Mouse 3 | 0.6 | 1.2 | 1.7 |
| Mouse 5 | 1.3 | 1.4 | 1.8 |
| Mouse 6 | 0.4 | 0.5 | 0.7 |
| Mouse 7 | 0.4 | 0.8 | 1 |
| Mouse 8 | 0.4 | 0.8 | 1.2 |
| Mouse 9 | 1 | 1.7 | 2.1 |
| Mouse 10 | 1 | 1.2 | 1.6 |
| Mouse 11 | 0.8 | 0.8 | 1.5 |
| Mouse 12 | 0.4 | 0.5 | 0.8 |
| Mouse 14 | 0.5 | 0.6 | 1.5 |

(continued)

|  | % donor type (dt) | % dt+ CD45+ | % dt+ CD3+ |
|---|---|---|---|
| Mouse 18 | 0.5 | 0.5 | 0.8 |

**Table 5: Peripheral Blood Analysis**

|  | % donor type (dt) | % dt+ CD45+ | % dt+ B220+ | % dt+ GR1+ | % dt+ CD11b+ | % dt+ CD3+ |
|---|---|---|---|---|---|---|
| mouse 2 | 16.7 | 20.6 | 1.4 | 4.5 | 89.2 | 1 |
| mouse 3 | 17.3 | 15.5 | 1.1 | 2.7 | 80 | 4.9 |
| mouse 5 | 17.6 | 17.3 | 0.8 | 5 | 87.3 | 0.8 |
| mouse 6 | 8.9 | 8.5 | 0.7 | 2 |  | 0.5 |
| mouse 7 | 17.9 | 17.5 | 1.3 | 4.5 |  | 0.7 |
| mouse 8 | 10.8 | 10.5 | 0.9 | 4 | 80.9 | 2.3 |
| mouse 9 | 12.9 | 13.3 | 0.9 | 2.7 |  | 1.1 |
| mouse 10 | 5 | 3.9 | 0.8 | 3.3 |  | 4.3 |
| mouse 11 | 9 | 8.8 | 0.8 | 4.2 |  | 0.8 |
| mouse 12 | 35.8 | 35.6 | 1.1 | 1.9 | 84.2 | 2.4 |
| mouse 14 | 9.7 | 9.4 | 0.8 | 3 |  | 0.4 |
| mouse 18 | 10.4 | 9.6 | 1.1 | 3.1 | 80.2 | 1.4 |
| Abbreviations:<br>dt cells of donor type CD45.2 (Ly 5.2)<br>CD45 pan-leukocyte marker for all white blood cells<br>c-kit marker of very early (pluripotent, reconstituting) stem cells<br>B220 marker for B cells of all maturation stages<br>Ter119 marker for all erythrocytic (red blood) stages<br>CD3 marker for all T lymphocytes including CD4 and CD8 positive cells<br>GR1 marker for granulocytes<br>CD11b commonly used as myeloid marker (white blood cells) but also detected on lymphoid cells | | | | | | |

**Example 6 RT-PCR for Cultured hMesSCs**

hMesSCs were cultured as described above for differentiation into HSC and endothelial cells. Cells were harvested for immunoflorescence as described above and for RT-PCR.

For RT-PCR, RNA was extracted using the Invisorb Spin Cell-RNA® Mini-kit (Invitek, Berlin, Germany) according to the manufacturer's instructions. RNA was stored at -80 °C. Reverse transcription (RT) of extracted RNA was performed using the bulk First-strand c-DNA synthesis kit (Amersham, Freiburg, Germany). The cDNA was stored at -20 °C. For the PCR reaction 5 μl cDNA-template was mixed with 2.5 μl 10 x PCR-buffer, 0.5 μl 10 mM dNTP's, 0.5 μl of each primer (50 ng/μl), and 0.5 μl polymerase (Ampli-Taq., Gibco) in a total volume of 25 μl for each probe. PCR was carried out in a programmable Biometra Uno-Thermobloc (Biometra, Göttingen, Germany) using the primers shown in Table 6. Negative controls were performed for each set of primers including water instead of cDNA. Samples were analyzed on 2 % agarose gels. The size of the PCR-fragments was estimated using the DNA-standard marker VIII (Gibco). Primers were synthesized by MWG Biotech (Ebersberg, Germany) according to the sequences in the Table 6.

Results of the RT-PCT showed gene expression detected using primers for CD41 B, Notch-1, GATA2, GATA3, Runx1, SCL, and CD117 (c-kit) that encode lineage restricted hematopoietic transcription factors. SCL, GATA-1 and GATA-2 are expressed in multipotent progenitors prior to lineage commitment, but are down-regulated during granulocyte/ monocyte differentiation RT-PCR results for cells up to 2 passages also showed weak bands for CD14 and CD34 which were not detected in cells in later passages.

**Table 6: RT-PCR Primers and Conditions**

| Marker | Product size | Primer | Sequence | SEQ ID NO |
|---|---|---|---|---|
| CD34 | 149 bp | F | CTG CTC CTT GCC CAG TCT G | 1 |
| | | R | GAA TAG CTC TGG TGG CTT GCA | 2 |
| CD133 | 591 bp | F | CCA CGA CTG TCG TAGCAG GT | 3 |
| | | R | GCT GTT CTG CAG GTG AAG AG | 4 |
| CD14 | 289 bp (619) | F1,2 | GCT GTT CTG CAG GTG AAG AG | 5 |
| | | R 1 | CCT CTA CTG CAGACA CAC A | 6 |
| | 619 bp | R 2 | GGC TTG AGC CAC TGC AGC | 7 |
| CD16 | 741 bp | F | CCT CAA TGG TAC AGC GTG C | 8 |
| | | R | GGG TTG CAA ATC CAG AGA AA | 9 |
| CD16 | 480 bp | F | GGA CAA TTC CAC ACA GTG G | 10 |
| | | R | GGA GTA CCA TCA CCA AGC A | 11 |
| CD41 B | 383 bp | F | GTC AGC TGG AGC GAC GTC A | 12 |
| | | R | GGA GTC AAA GGA GAG GCT C | 13 |
| Glycophorin A | 884 bp | F | CCT TCA TTC TGA ACA GGC AA | 14 |
| | | R | CCT TGG CAT TTG GGT CAT T | 15 |
| VWF | 438 bp | F 1 | GAG TGA GCC TCT CCG TGT A | 16 |
| | | R 1 | GCT CTT CAG AAG CTG GCA C | 17 |
| | 263 bp (664) | F 2 (3) | CCA GAT TTG CCA CTG TGA TG | 18 |
| | | R2 | CCA CTT CTG GGA GAT GCG CA | 19 |
| | | R3 | CCA CAC TGC TCA GCA CGA AG | 20 |
| NOTCH-1 | 177 bp | F | GCA CTG CGA GGT CAA CAC | 21 |
| | | R | AGG CAC TTG GCA CCA TTC | 22 |
| GATA1 | 173 bp | F | CAG AAA CGC CGA GGG TGA | 23 |
| | | R | TTA GAA GAG GTG GAA GTT GGA GTC A | 24 |
| GATA2 | 182 bp | F | ACA ACC ACC ACC TTA TGG CG | 25 |
| | | R | GCA TGC ACT TTG ACA GCT CC | 26 |
| GATA3 | 573 bp | F | CCT GCA GTC CCT TTC GAC | 27 |
| | | R | GCA ACT GGT GAA CGG TAA | 28 |
| GATA4 | 612 bp | F | CGA CTT CTC AGA AGG CAGA | 29 |
| | | R | GGG AGA CGC ATA GCC TTG T | 30 |
| Runx-1 | 403 bp | F | GGT CAA CTC AGT TCC AGA G | 31 |
| | | R | CTG GTG CAC AGG TAA AAG | 32 |
| Tal1 (SCL) | 386 bp | F | GGA GCA AAC ACA GTT GGA T | 33 |
| | | R | GGC ATC AGA GAC TGT GCT T | 34 |
| CD117 c-kit | 486 bp | F | GGA GAT CTG TGA GAA TAG GCT | 35 |
| | | R | CAT ACA TTT CAG CAG GTG CG | 36 |
| KDR | 626 bp | F | TAC TTG TCC ATC GTC ATG GAT CCA G | 37 |

EP 1 673 445 B1

(continued)

| Marker | Product size | Primer | Sequence | SEQ ID NO |
|---|---|---|---|---|
| | | R | CTG TAA CAG ATG AGA TGC TCC AAG G | 38 |
| Hemoglobin alpha 1 | 195 bp | F | CCA CAG ACT CAG AGA GAA C | 39 |
| | | R | CCT TAA CCT GGG CAG AGC | 40 |
| Hemoglobin alpha 2 | 212 bp | F | CTG GAG AGG ATG TTC CTG T | 41 |
| | | R | GCT TGA AGT TGA CCG GGT | 42 |
| c-MPL | 666 bp | F | CCT GTA TAT AAT CCT CAC CAA | 43 |
| | | R | GCT CAT CTG CAG GCA CTG | 44 |
| EPOR | 311 bp | F | GCT GTA TCA TGG ACC ACC T | 45 |
| | | R | GCT TCC ATG GCT CAT CCT | 46 |
| CD31 | 654 bp | F | GGA GTT TCC AGA AAT CAT AA | 47 |
| | | R | GGA ATG GCA ATT ATC TGC AA | 48 |

**Claims**

1. A method of producing blood products *in vitro,* in which

   a) non-SV40 transformed mesenchymal stem cells are isolated, which are CD34-negative, and which are negative for CD45 and positive for CD105, CD59, CD90, CD13, and MHC I after at least one passage in culture; and
   b) said isolated non-SV40 transformed mesenchymal stem cells are cultured with the following growth factors added individually or in combinations thereof:
   stem cell factor (SCF), thrombopoietin (TPO), flt-3 ligand (FL), interleukins, including interleukin-3 (IL-3) and interleukin-6 (IL-6), granulocyte-colony stimulating factor (G-CSF), granulocyte-macrophage-colony stimulating factor (GM-CSF), erythropoietin (Epo), vascular-endothelial growth factor (VEGF), basic fibroblast growth factor (bFGF), hepatocyte growth factor (HGF), insulin-like growth factor (IGF), epidermal growth factor (EGF), leukaemia inhibitory factor (LIF), and hydrocortisone (HC),
   for a time sufficient to produce at least one type of blood products.

2. The method of claim 1 wherein said at least one type of blood products comprises myeloid stem cells.

3. The method of claim 1 wherein said at least one type of blood products comprises endothelial cells.

4. The method of claim 1 wherein said at least one type of blood products comprises lymphoid stem cells.

5. The method of claim 1 wherein said at least one type of blood products comprises dendritic cells.

6. The method of claim 1 wherein said at least one type of blood products comprises erythroid cells.

7. The method of claim 1 wherein said at least one type of blood products comprises megakaryocytes.

8. Use of a blood product obtained according to a method of any one of claims claims 1 to 7 for preparing a pharmaceutical composition for treating a patient in need of a blood product.

9. The use of claim 8 wherein said at least one type of blood products comprises myeloid stem cells.

10. The use of claim 8 wherein said at least one type of blood products comprises endothelial cells.

11. The use of claim 8 wherein said at least one type of blood products comprises lymphoid stem cells.

12. The use of claim 8 wherein said at least one type of blood products comprises dendritic cells.

13. The use of claim 8 wherein said at least one type of blood products comprises erythroid cells.

14. The use of claim 8 wherein said at least one type of blood products comprises megakaryocytes.

15. The use of claim 8, wherein the pharmaceutical composition is for treating patients suffering from leukemia, thrombocytopenia, leukopenia, granulocytopenia, lymphocytopenia, aplastic anemia, and/or autoimmune disease with or without bone marrow involvement, HIV patients, patients after chemotherapy, total body irradiation or irradiation of single parts of the body, patients with vascular, ischemic and/or malignant disease, or patients with cardiac ischemia.

16. The use of claim 8, wherein the the pharmaceutical composition is for treating patients suffering from anemia, leukopenia, thrombocytopenia and vascular diseases.

17. The use of claim 16, wherein the anemia is due to acute leukemia, due to chronic leukemia, due to osteomyelofibrosis, due to aplastic anemia, due to thalassaemia, due to sickle cell disease, due to loss of blood, due to chemotherapy, due to medical drugs other than chemotherapy, due to radiation, and/or due to abuse of toxic compounds, wherein the leukopenia is due to acute leukemia, due to chronic leukemia, due to osteomyelofibrosis, due to aplastic anemia, due to thalassaemia, due to sickle cell disease, due to loss of blood, e.g. after an accident, due to chemotherapy, due to medical drugs other than chemotherapy, due to radiation, and/or due to abuse of toxic compounds, wherein the thrombocytopenia is due to acute leukemia, due to chronic leukemia, due to osteomyelofibrosis, due to aplastic anemia, due to thalassaemia, due to sickle cell disease, due to loss of blood, e.g. after an accident, due to chemotherapy, due to medical drugs other than chemotherapy, due to radiation, and/or due to abuse of toxic compounds.

18. The use of claim 16, wherein the vascular disease is autoimmune vasculitis, arterial occlusive disorders, venous occlusive disease, and/or artherosclerosis, and for treating of patients suffering from ischemic diseases, such as coronary heart disease, stroke, acute renal failure, and/or claudicatio intermittens.

19. A method of differentiating non-SV40 transformed mesenchymal cells *in vitro,* in which

   a) non-SV40 transformed mesenchymal stem cells are isolated, which are CD34-negative, and which are negative for CD45 and positive for CD105, CD59, CD90, CD13, and MHC I after at least one passage in culture; and
   b) said isolated non-SV40 transformed mesenchymal stem cells are cultured with the following growth factors added individually or in combinations thereof:

   stem cell factor (SCF), thrombopoietin (TPO), flt-3 ligand (FL), interleukins, including interleukin-3 (IL-3) and interleukin-6 (IL-6), granulocyte-colony stimulating factor (G-CSF), granulocyte-macrophage-colony stimulating factor (GM-CSF), erythropoietin (Epo), vascular-endothelial growth factor (VEGF), basic fibroblast growth factor (bFGF), hepatocyte growth factor (HGF), insulin-like growth factor (IGF), epidermal growth factor (EGF), leukaemia inhibitory factor (LIF), and hydrocortisone (HC),
for a time sufficient to produce at least one type of blood products.

20. The method of claim 19 wherein said at least one type of blood products comprises myeloid stem cells.

21. The method of claim 19 wherein said at least one type of blood products comprises endothelial cells.

22. The method of claim 19 wherein said at least one type of blood products comprises lymphoid stem cells.

23. The method of claim 19 wherein said at least one type of blood products comprises dendritic cells.

24. The method of claim 19 wherein said at least one type of blood products comprises erythroid cells.

25. The method of claim 19 wherein said at least one type of blood products comprises megakaryocytes.

26. Method for preparing a pharmaceutical composition comprising a blood product for treating a patient in need of said blood product, wherein said method comprises the method of any one of claims 1 to 7 for providing said blood product.

27. The method of claim 26 wherein said at least one type of blood products comprises myeloid stem cells.

28. The method of claim 26 wherein said at least one type of blood products comprises endothelial cells.

29. The method of claim 26 wherein said at least one type of blood products comprises lymphoid stem cells.

30. The method of claim 26 wherein said at least one type of blood products comprises dendritic cells.

31. The method of claim 26 wherein said at least one type of blood products comprises erythroid cells.

32. The method of claim 26 wherein said at least one type of blood products comprises megakaryocytes.

33. The method of claim 26, wherein the pharmaceutical composition is for treating patients suffering from leukemia, thrombocytopenia, leukopenia, granulocytopenia, lymphocytopenia, aplastic anemia, and/or autoimmune disease with or without bone marrow involvement, HIV patients, patients after chemotherapy, total body irradiation or irradiation of single parts of the body, patients with vascular, ischemic and/or malignant disease, or patients with cardiac ischemia.

34. The method of claim 26, wherein the the pharmaceutical composition is for treating patients suffering from anemia, leukopenia, thrombocytopenia and vascular diseases.

35. The method of claim 34, wherein the anemia is due to acute leukemia, due to chronic leukemia, due to osteomyelofibrosis, due to aplastic anemia, due to thalassaemia, due to sickle cell disease, due to loss of blood, due to chemotherapy, due to medical drugs other than chemotherapy, due to radiation, and/or due to abuse of toxic compounds, wherein the leukopenia is due to acute leukemia, due to chronic leukemia, due to osteomyelofibrosis, due to aplastic anemia, due to thalassaemia, due to sickle cell disease, due to loss of blood, e.g. after an accident, due to chemotherapy, due to medical drugs other than chemotherapy, due to radiation, and/or due to abuse of toxic compounds, wherein the thrombocytopenia is due to acute leukemia, due to chronic leukemia, due to osteomyelofibrosis, due to aplastic anemia, due to thalassaemia, due to sickle cell disease, due to loss of blood, e.g. after an accident, due to chemotherapy, due to medical drugs other than chemotherapy, due to radiation, and/or due to abuse of toxic compounds.

36. The method of claim 16, wherein the vascular disease is autoimmune vasculitis, arterial occlusive disorders, venous occlusive disease, and/or artherosclerosis, and for treating of patients suffering from ischemic diseases, such as coronary heart disease, stroke, acute renal failure, and/or claudicatio intermittens.

**Patentansprüche**

1. Verfahren zur Herstellung von Blutprodukten in vitro, bei dem

a) nicht-SV40-transformierte mesenchymale Stammzellen isoliert werden, die CD34-negativ sind, und die negativ für CD45 und positiv für CD105, CD59, CD90, CD13 und MHC I nach mindestens einer Passage in Kultur sind; und

b) die isolierten nicht-SV40-transformierten mesenchymalen Stammzellen mit den folgenden Wachstumsfaktoren kultiviert werden, die individuell oder in Kombinationen derselben zugefügt werden:

Stammzellfaktor (SCF), Thrombopoietin (TPO), Flt-3 Ligand (FL), Interleukine, einschließlich Interleukin-3 (IL-3) und Interleukin-6 (IL-6), Granulozyten-Kolonie stimulierender Faktor (G-CSF), Granulozyten-Makrophagen-Kolonie stimulierender Faktor (GM-CSF), Erythropioetin (Epo), Vaskulär-endothelialer Wachstumsfaktor (VEGF), basischer Fibroblasten Wachstumsfaktor (bFGF), Hepatozyten Wachstumsfaktor (HGF), Insulin-ähnlicher Wachstumsfaktor (IGF), epidermaler Wachstumsfaktor (EGF), Leukämie inhibitorischer Faktor (LIF) und Hydrocortison (HC), für eine Zeit, die ausreicht, um mindestens eine Art von Blutprodukten zu bilden.

2. Verfahren nach Anspruch 1, bei dem die mindestens eine Art von Blutprodukten myeloide Stammzellen umfasst.

3. Verfahren nach Anspruch 1, bei dem die mindestens eine Art von Blutprodukten endotheliale Zellen umfasst.

4. Verfahren nach Anspruch 1, bei dem die mindestens eine Art von Blutprodukten lymphoide Stammzellen umfasst.

5. Verfahren nach Anspruch 1, bei dem die mindestens eine Art von Blutprodukten dendritische Zellen umfasst.

6. Verfahren nach Anspruch 1, bei dem die mindestens eine Art von Blutprodukten erythroide Zellen umfasst.

7. Verfahren nach Anspruch 1, bei dem die mindestens eine Art von Blutprodukten Megakaryozyten umfasst.

8. Verwendung eines Blutprodukts, erhalten nach einem Verfahren nach irgendeinem der Ansprüche 1 bis 7 zur Herstellung einer pharmazeutischen Zusammensetzung zur Behandlung eines Patienten, der ein Blutprodukt benötigt.

9. Verwendung nach Anspruch 8, bei dem die mindestens eine Art von Blutprodukten myeloide Stammzellen umfasst.

10. Verwendung nach Anspruch 8, bei dem die mindestens eine Art von Blutprodukten endotheliale Zellen umfasst.

11. Verwendung nach Anspruch 8, bei dem die mindestens eine Art von Blutprodukten lyphoide Stammzellen umfasst.

12. Verwendung nach Anspruch 8, bei dem die mindestens eine Art von Blutprodukten dendritische Zellen umfasst.

13. Verwendung nach Anspruch 8, bei dem die mindestens eine Art von Blutprodukten erythroide Zellen umfasst.

14. Verwendung nach Anspruch 8, bei dem die mindestens eine Art von Blutprodukten Megakaryozyten umfasst.

15. Verwendung nach Anspruch 8, bei dem die pharmazeutische Zusammensetzung zur Behandlung von Patienten ist, die an Leukämie, Thrombozytopenie, Leukopenie, Granulozytopenie, Lymphozytopenie, aplastischer Anämie und/oder Autoimmunerkrankung mit oder ohne Knochenmarkbeteiligung leiden, von HIV-Patienten, Patienten nach Chemotherapie, Gesamtkörperbestrahlung oder Bestrahlung von einzelnen Teilen des Körpers, von Patienten mit vaskulärer, ischämischer und/oder maligner Erkrankung oder von Patienten mit kardiärer Ischämie.

16. Verwendung nach Anspruch 8, bei dem die pharmazeutische Zusammensetzung für die Behandlung von Patienten ist, die an Anämie, Leukopenie, Thrombozytopenie und vaskluären Erkrankungen leiden.

17. Verwendung nach Anspruch 16, bei dem die Anämie auf akuter Leukämie beruht, auf chronischer Leukämie beruht, auf Osteomyelofibrose beruht, auf aplastischer Anämie beruht, auf Thalassämie beruht, auf Sichelzellerkrankung beruht, auf Blutverlust beruht, auf Chemotherapie beruht, auf anderen medizinischen Wirkstoffen als Chemotherapie beruht, auf Bestrahlung beruht und/oder auf Missbrauch toxischer Verbindungen beruht, wobei die Leukopenie auf akuter Leukämie beruht, auf chronischer Leukämie beruht, auf Osteomyelofiabrose beruht, auf aplastischer Anämie beruht, auf Thalassämie beruht, auf Sichelzellerkrankungen beruht, auf Blutverlust beruht, wie nach einem Unfall, auf Chemotherapie beruht, auf anderen medizinischen Wirkstoffen als Chemotherapie beruht, auf Bestrahlung beruht und/oder auf den Missbrauch von toxischen Verbindungen beruht, wobei die Thrombozytopenie auf akute Leukämie beruht, auf chronischer Leukämie beruht, auf Osteomyelofibrose beruht, auf aplastischer Anämie beruht, auf Thalassämie beruht, auf Sichelzellerkrankungen beruht, auf Blutverlust beruht, wie nach einem Unfall, auf Chemotherapie beruht, auf anderen medizinischen Wirkstoffen als Chemotherapie beruht, auf Bestrahlung beruht und/oder auf dem Missbrauch von toxischen Verbindungen beruht.

18. Verwendung nach Anspruch 16, bei dem die vaskuläre Erkrankung eine autoimmune Vaskulitis, arterielle okklusive Erkrankungen, venöse okklusive Erkrankung, und/oder Arteriosklerose ist und zur Behandlung von Patienten, die an ischämischen Erkrankungen, wie z.B. koronarer Herzerkrankungen, Schlaganfall, akutem Nierenversagen und/oder Claudicatio intermittens leiden.

19. Verfahren zum Differenzieren von nicht-SV40-transformierten mesenchymalen Zellen in vitro, bei dem

a) nicht-SV40-transformierte mesenchymale Stammzellen isoliert werden, die CD34-negativ sind, und die negativ für CD45 und positiv für CD105, CD59, CD90, CD13 und MHC I nach mindestens einer Passage in Kultur sind; und
b) die isolierten nicht-SV40-transformierten mesenchymalen Stammzellen mit den folgenden Wachstumsfaktoren kultiviert werden, die individuell oder in Kombinationen derselben zugefügt werden:

Stammzellfaktor (SCF), Thrombopoietin (TPO), Flt-3 Ligand (FL), Interleukine, einschließlich Interleukin-3 (IL-3) und Interleukin-6 (IL-6), Granulozyten-Kolonie stimulierender Faktor (G-CSF), Granulozyten-Makrophagen-Kolonie stimulierender Faktor (GM-CSF), Erythropioetin (Epo), Vaskulär-endothelialer Wachstumsfaktor (VEGF), basischer Fibroblasten Wachstumsfaktor (bFGF), Hepatozyten Wachstumsfaktor (HGF), Insulin-ähnlicher Wachstumsfaktor (IGF), epidermaler Wachstumsfaktor (EGF), Leukämie inhibitorischer Faktor (LIF) und Hydrocortison (HC),
für eine Zeit, die ausreicht, um mindestens eine Art von Blutprodukten.

20. Verfahren nach Anspruch 19, bei dem die mindestens eine Art von Blutprodukten myeloide Stammzellen umfasst.

21. Verfahren nach Anspruch 19, bei dem die mindestens eine Art von Blutprodukten endotheliale Zellen umfasst.

22. Verfahren nach Anspruch 19, bei dem die mindestens eine Art von Blutprodukten lymphoide Stammzellen umfasst.

23. Verfahren nach Anspruch 19, bei dem die mindestens eine Art von Blutprodukten dendritische Zellen umfasst.

24. Verfahren nach Anspruch 19, bei dem die mindestens eine Art von Blutprodukten erythroide Zellen umfasst.

25. Verfahren nach Anspruch 19, bei dem die mindestens eine Art von Blutprodukten Megakaryozyten umfasst.

26. Verfahren zur Herstellung einer pharmazeutischen Zusammensetzung, die ein Blutprodukt umfasst, zur Behandlung eines Patienten, der dieses Blutprodukt benötigt, wobei das Verfahren das Verfahren nach irgendeinem der Ansprüche 1 bis 7 zur Bereitstellung dieses Blutprodukts umfasst.

27. Verfahren nach Anspruch 26, bei dem die mindestens eine Art von Blutprodukten myeloide Stammzellen umfasst.

28. Verfahren nach Anspruch 26, bei dem die mindestens eine Art von Blutprodukten endotheliale Zellen umfasst.

29. Verfahren nach Anspruch 26, bei dem die mindestens eine Art von Blutprodukten lymphoide Stammzellen umfasst

30. Verfahren nach Anspruch 26, bei dem die mindestens eine Art von Blutprodukten dendritische Zellen umfasst.

31. Verfahren nach Anspruch 26, bei dem die mindestens eine Art von Blutprodukten erythroide Zellen umfasst.

32. Verfahren nach Anspruch 26, bei dem die mindestens eine Art von Blutprodukten Megakaryozyten umfasst.

33. Verfahren nach Anspruch 26, bei dem die pharmazeutische Zusammensetzung zur Behandlung von Patienten ist, die an Leukämie, Thrombozytopenie, Leukopenie, Granulozytopenie, Lymphozytopenie, aplastischer Anämie und/oder Autoimmunerkrankung mit oder ohne Knochenmarkbeteiligung leiden, von HIV-Patienten, Patienten nach Chemotherapie, Gesamtkörperbestrahlung oder Bestrahlung von einzelnen Teilen des Körpers, von Patienten mit vaskulärer, ischämischer und/oder maligner Erkrankung oder von Patienten mit kardiärer Ischämie.

34. Verfahren nach Anspruch 26, bei dem die pharmazeutische Zusammensetzung für die Behandlung von Patienten ist, die an Anämie, Leukopenie, Thrombozytopenie und vaskluären Erkrankungen leiden.

35. Verfahren nach Anspruch 34, bei dem die Anämie auf akuter Leukämie beruht, auf chronischer Leukämie beruht, auf Osteomyelofibrose beruht, auf aplastischer Anämie beruht, auf Thalassämie beruht, auf Sichelzellerkrankung beruht, auf Blutverlust beruht, auf Chemotherapie beruht, auf anderen medizinischen Wirkstoffen als Chemotherapie beruht, auf Bestrahlung beruht und/oder auf Missbrauch toxischer Verbindungen beruht, wobei die Leukopenie auf akuter Leukämie beruht, auf chronischer Leukämie beruht, auf Osteomyelofiabrose beruht, auf aplastischer Anämie beruht, auf Thalassämie beruht, auf Sichelzellerkrankungen beruht, auf Blutverlust beruht, wie nach einem Unfall, auf Chemotherapie beruht, auf anderen medizinischen Wirkstoffen als Chemotherapie beruht, auf Bestrahlung beruht und/oder auf den Missbrauch von toxischen Verbindungen beruht, wobei die Thrombozytopenie auf akute Leukämie beruht, auf chronischer Leukämie beruht, auf Osteomyelofibrose beruht, auf aplastischer Anämie beruht, auf Thalassämie beruht, auf Sichelzellerkrankungen beruht, auf Blutverlust beruht, wie nach einem Unfall, auf Chemotherapie beruht, auf anderen medizinischen Wirkstoffen als Chemotherapie beruht, auf Bestrahlung beruht und/oder auf dem Missbrauch von toxischen Verbindungen beruht.

36. Verfahren nach Anspruch 16, bei dem die vaskuläre Erkrankung eine autoimmune Vaskulitis, arterielle okklusive Erkrankungen, venöse okklusive Erkrankung, und/oder Arteriosklerose ist und zur Behandlung von Patienten, die an ischämischen Erkrankungen, wie z.B. koronarer Herzerkrankungen, Schlaganfall, akutem Nierenversagen und/oder Claudicatio intermittens leiden.

## Revendications

1. Procédé de production de produits sanguins *in vitro,* dans lequel

   a) des cellules souches mésenchymateuses non transformées par SV40 sont isolées, qui sont CD34-négatives, et qui sont négatives pour CD45 et positives pour CD105, CD59, CD90, CD13, et CMH I après au moins un passage en culture ; et
   b) lesdites cellules mésenchymateuses non transformées par SV40 isolées sont cultivées avec les facteurs de croissance suivants ajoutés individuellement ou en combinaisons de plusieurs d'entre eux :

   facteur des cellules souches (FCS), thrombopoïétine (TPO), ligand de flt-3 (FL), interleukines, incluant l'interleukine-3 (IL-3) et l'interleukine-6 (IL-6), facteur stimulant les colonies de granulocytes (G-CSF), facteur stimulant les colonies de granulocytes et de macrophages (GM-CSF), erythropoïétine (Epo), facteur de croissance vasculaire-endothéliale (VEGF), facteur de croissance des fibroblastes basique (bFGF), facteur de croissance des hépatocytes (HGF), facteur de croissance analogue à l'insuline (IGF), facteur de croissance épidermique (EGF), facteur inhibiteur de la leucémie (FIL) et hydrocortisone (HC), pendant une durée suffisante pour produire au moins un type de produit sanguin.

2. Procédé de la revendication 1, dans lequel ledit au moins un type de produit sanguin comprend des cellules souches myéloïdes.

3. Procédé de la revendication 1, dans lequel ledit au moins un type de produit sanguin comprend des cellules endothéliales.

4. Procédé de la revendication 1, dans lequel ledit au moins un type de produit sanguin comprend des cellules souches lymphoïdes.

5. Procédé de la revendication 1, dans lequel ledit au moins un type de produit sanguin comprend des cellules dendritiques.

6. Procédé de la revendication 1, dans lequel ledit au moins un type de produit sanguin comprend des cellules érythroïdes.

**7.** Procédé de la revendication 1, dans lequel ledit au moins un type de produit sanguin comprend des mégacaryocytes.

**8.** Utilisation d'un produit sanguin obtenu selon un procédé de l'une quelconque des revendications 1 à 7 pour préparer une composition pharmaceutique pour traiter un patient nécessitant un produit sanguin.

**9.** Utilisation de la revendication 8, dans laquelle ledit au moins un type de produit sanguin comprend des cellules souches myéloïdes.

**10.** Utilisation de la revendication 8, dans laquelle ledit au moins un type de produit sanguin comprend des cellules endothéliales.

**11.** Utilisation de la revendication 8, dans laquelle ledit au moins un type de produit sanguin comprend des cellules souches lymphoïdes.

**12.** Utilisation de la revendication 8, dans laquelle ledit au moins un type de produit sanguin comprend des cellules dendritiques.

**13.** Utilisation de la revendication 8, dans laquelle ledit au moins un type de produit sanguin comprend des cellules érythroïdes.

**14.** Utilisation de la revendication 8, dans laquelle ledit au moins un type de produit sanguin comprend des mégacaryocytes.

**15.** Utilisation de la revendication 8, dans laquelle la composition pharmaceutique est destinée au traitement de patients souffrant de leucémie, thrombocytopénie, leucopénie, granulocytopénie, lymphocytopénie, anémie aplasique, et/ou maladie auto-immune avec ou sans implication de la moelle osseuse, de patients VIH, de patients après chimiothérapie, irradiation de l'organisme entier ou irradiation de parties individuelles de l'organisme, de patients atteints de maladies vasculaires, ischémiques et/ou malignes ou de patients atteints d'ischémie cardiaque.

**16.** Utilisation de la revendication 8, dans laquelle la composition pharmaceutique est destinée au traitement de patients souffrant d'anémie, de leucopénie, de thrombocytopénie, et de maladies vasculaires.

**17.** Utilisation de la revendication 16, dans laquelle l'anémie est due à une leucémie aiguë, due à une leucémie chronique, due à une ostéomyélofibrose, due à une aplasie anémique, due à une thalassémie, due à une drépanocytose, due à une perte de sang, due à une chimiothérapie, due à des substances médicamenteuses hors chimiothérapie, due à une irradiation, et/ou due à un abus de substances toxiques, dans laquelle la leucopénie est due à une leucémie aiguë, due à une leucémie chronique, due à une ostéomyélofibrose, due à une aplasie anémique, due à une thalassémie, due à une drépanocytose, due à une perte de sang, par exemple après un accident, due à une chimiothérapie, due à des substances médicamenteuses hors chimiothérapie, due à une irradiation, et/ou due à un abus de substances toxiques, dans laquelle la thrombocytopénie est due à une leucémie aiguë, due à une leucémie chronique, due à une ostéomyélofibrose, due à une aplasie anémique, due à une thalassémie, due à une drépanocytose, due à une perte de sang, par exemple après un accident, due à une chimiothérapie, due à des substances médicamenteuses hors chimiothérapie, due à une irradiation, et/ou due à un abus de substances toxiques.

**18.** Utilisation de la revendication 16, dans laquelle la maladie vasculaire est une vasculite auto-immune, des affections artérielles occlusives, une maladie veineuse occlusive et/ou un arthérosclérose et pour traiter des patients souffrant de maladies ischémiques, telles qu'une maladie coronarienne, une attaque, une insuffisance rénale aiguë et/ou une claudication intermittente.

**19.** Procédé de différenciation de cellules mésenchymateuses non transformées par SV40 *in vitro,* dans lequel

a) des cellules souches mésenchymateuses non transformées par SV40 sont isolées, qui sont CD34-négatives, et qui sont négatives pour CD45 et positives pour CD105, CD59, CD90, CD13, et CMH I après au moins un passage en culture ; et

b) lesdites cellules mésenchymateuses non transformées par SV40 isolées sont cultivées avec les facteurs de croissance suivants ajoutés individuellement ou en combinaisons de plusieurs d'entre eux :

facteur des cellules souches (FCS), thrombopoïétine (TPO), ligand de flt-3 (FL), interleukines, incluant l'interleukine-3 (IL-3) et l'interleukine-6 (IL-6), facteur stimulant les colonies de granulocytes (G-CSF), facteur stimulant les colonies de granulocytes et de macrophages (GM-CSF), erythropoïétine (Epo), facteur de croissance vasculaire-endothéliale (VEGF), facteur de croissance des fibroblastes basique (bFGF), facteur de croissance des hépatocytes (HGF), facteur de croissance analogue à l'insuline (IGF), facteur de croissance épidermique (EGF), facteur inhibiteur de la leucémie (FIL) et hydrocortisone (HC), pendant une durée suffisante pour produire au moins un type de produit sanguin.

20. Procédé de la revendication 19, dans lequel ledit au moins un type de produit sanguin comprend des cellules souches myéloïdes.

21. Procédé de la revendication 19, dans lequel ledit au moins un type de produit sanguin comprend des cellules endothéliales.

22. Procédé de la revendication 19, dans lequel ledit au moins un type de produit sanguin comprend des cellules souches lymphoïdes.

23. Procédé de la revendication 19, dans lequel ledit au moins un type de produit sanguin comprend des cellules dendritiques.

24. Procédé de la revendication 19, dans lequel ledit au moins un type de produit sanguin comprend des cellules érythroïdes.

25. Procédé de la revendication 19, dans lequel ledit au moins un type de produit sanguin comprend des mégacaryocytes.

26. Procédé pour préparer une composition pharmaceutique comprenant un produit sanguin pour traiter un patient nécessitant ledit produit sanguin, lequel dit procédé comprend le procédé selon l'une quelconque des revendications 1 à 7 pour fournir ledit produit sanguin.

27. Procédé de la revendication 26, dans lequel ledit au moins un type de produit sanguin comprend des cellules souches myéloïdes.

28. Procédé de la revendication 26, dans lequel ledit au moins un type de produit sanguin comprend des cellules endothéliales.

29. Procédé de la revendication 26, dans lequel ledit au moins un type de produit sanguin comprend des cellules souches lymphoïdes.

30. Procédé de la revendication 26, dans lequel ledit au moins un type de produit sanguin comprend des cellules dendritiques.

31. Procédé de la revendication 26, dans lequel ledit au moins un type de produit sanguin comprend des cellules érythroïdes.

32. Procédé de la revendication 26, dans lequel ledit au moins un type de produit sanguin comprend des mégacaryocytes.

33. Procédé de la revendications 26, dans lequel la composition pharmaceutique est destinée au traitement de patients souffrant de leucémie, thrombocytopénie, leucopénie, granulocytopénie, lymphocytopénie, anémie aplasique, et/ou maladie auto-immune avec ou sans implication de la moelle osseuse, de patients VIH, de patients après chimiothérapie, irradiation de l'organisme entier ou irradiation de parties individuelles de l'organisme, de patients atteints de maladies vasculaires, ischémiques et/ou malignes ou de patients atteints d'ischémie cardiaque.

34. Procédé de la revendication 26, dans lequel la composition pharmaceutique est destinée au traitement de patients souffrant d'anémie, de leucopénie, de thrombocytopénie, et de maladies vasculaires.

35. Procédé de la revendication 34, dans lequel l'anémie est due à une leucémie aiguë, due à une leucémie chronique, due à une ostéomyélofibrose, due à une aplasie anémique, due à une thalassémie, due à une drépanocytose, due à une perte de sang, due à une chimiothérapie, due à des substances médicamenteuses hors chimiothérapie, due à une irradiation, et/ou due à un abus de substances toxiques, dans lequel la leucopénie est due à une leucémie aiguë, due à une leucémie chronique, due à une ostéomyélofibrose, due à une aplasie anémique, due à une thalassémie, due à une drépanocytose, due à une perte de sang, par exemple après un accident, due à une chimiothérapie, due à des substances médicamenteuses hors chimiothérapie, due à une irradiation, et/ou due à un abus de substances toxiques, dans lequel la thrombocytopénie est due à une leucémie aiguë, due à une leucémie chronique, due à une ostéomyélofibrose, due à une aplasie anémique, due à une thalassémie, due à une drépanocytose, due à une perte de sang, par exemple après un accident, due à une chimiothérapie, due à des substances médicamenteuses hors chimiothérapie, due à une irradiation, et/ou due à un abus de substances toxiques.

36. Procédé de la revendication 16, dans lequel la maladie vasculaire est une vasculite auto-immune, des affections artérielles occlusives, une maladie veineuse occlusive et/ou un arthérosclérose et pour traiter des patients souffrant de maladies ischémiques, telles qu'une maladie coronarienne, une attaque, une insuffisance rénale aiguë et/ou une claudication intermittente.

**Figure 1**

**Figure 2:** Growth and doubling of MSC in vitro

**Figure 3:** Blood cell counts after transplantation with mMesSCs

**Figure 4:** Analysis of subpopulation of transplanted mice
Abbreviations: dt, donor type.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9961588 A
- DE 10336152
- EP 2004008865 W
- US 60510980 B

**Non-patent literature cited in the description**

- **PITTINGER, MF et al.** *Science,* 1999, vol. 248, 385-389
- **JAISWAL, N et al.** *J. Cell Biochem.,* 1997, vol. 64 (2), 295-312
- **SHAKIBAEI, M et al.** *Cell Biol. Int,* 1997, vol. 21 (2), 115-25
- **FUKUDA, K.** *Artif. Organs,* 2001, vol. 25 (3), 187-93
- **DENG et al.** *Biochem. Biophys. Res. Commun,* 2001, vol. 282 (1), 148-52
- **WOODBURY et al.** *J. Neurosci. Res,* 2000, vol. 61 (4), 364-70
- **TOMA, C et al.** *Circulation,* 2002, vol. 105 (1), 93-8
- **HOFSTETTER et al.** *Proc. Natl. Acad. Sci. U.S.A.,* 1999, vol. 99 (4), 2199-204